# EUROPÄISCHE PATENTSCHRIFT

(11) **EP 0 371 067 B1**
(45) Veröffentlichungstag und Bekanntmachung des Hinweises auf die Patenterteilung: **03.03.1993**
(21) Anmeldenummer: 88907038.9
(22) Anmeldetag: 21.07.1988
(51) Int. Cl.: C12N 15/10, C12Q 1/68, C12M 1/34

(54) **VERFAHREN UND VORRICHTUNG ZUR REINIGUNG VON M-13-PHAGEN-DNA**
PROCESS AND DEVICE FOR PURIFICATION OF M-13 PHAGE DNA
PROCEDE ET DISPOSITIF DE PURIFICATION D'ADN DE PHAGES M-13

(30) Priorität: 23.07.1987 DE 3724442
(43) Veröffentlichungstag der Anmeldung: 06.06.1990
(73) Patentinhaber: EUROPÄISCHES LABORATORIUM FÜR MOLEKULARBIOLOGIE (EMBL), D-69117 Heidelberg (DE)
(72) Erfinder: ANSORGE, Wilhelm, D-6901 Gaiberg b. Heidelberg (DE); KRISTENSEN, Tom, N-0470 Oslo 4 (NO); VOSS, Hartmut, D-6901 Gaiberg (DE); STEGEMANN, Josef, D-6900 Heidelberg (DE); SCHWAGER, Christian, D-6703 Limburgerhof (DE)
(74) Vertreter: Huber, Bernhard, Dipl.-Chem.
(86) Internationale Anmeldenummer: EP8800659
(87) Internationale Veröffentlichungsnummer: WO8901035

(56) Entgegenhaltungen:
- WO-A-85/05631
- WO-A-87/02132
- GB-A- 2 139 349
- Chemical abstracts, vol. 90, 1979, (Columbus, Ohio, US), see abstract n 2778s & Exp. Cell. Res. 1978, 115(2), 411-14
- Analytical biochemistry, vol. 101, 1980, C.W. Chen et al. "Recovery of DNA segments from agarose gels", pages 339-341

## Beschreibung

Bei der DNA-Sequenzierung nach Sanger (Proc.Natl.Acad. Sci. USA, 74, 54-63 (1977)) werden von jeder der vier in der DNA vorkommenden Basen 2',3'-Didesoxynucleotidtriphosphate hergestellt, welche von der DNA-Polymerase in einen wachsenden DNA-Strang zwar eingebaut werden, aber mit dem darauf folgenden Desoxynucleotidphosphat keine Phosphodiesterbrücke auszubilden vermögen, so daß die Kette nicht mehr weiterwächst. Wird zur Sequenzierung der zu analysierende DNA-Strang mit einem kurzen Stück markierter DNA (dem Primer) die dem einen Ende des zu analysierenden Stranges komplementär ist, einem der Didesoxynucleotidtriphosphate und den drei anderen normalen Triphosphaten in Gegenwart von DNA-Polymerase umgesetzt, so wird vom Primer ausgehend ein komplementärer Strang zur zu bestimmenden DNA synthetisiert, dessen Kette dort abbricht, wo eine Didesoxyverbindung eingebaut wird. Man erhält so in vier Ansätzen mit jeweils einem der vier Didesoxynucleotidtriphosphate eine Reihe markierter Stränge, die der Größe nach durch Gelchromatographie aufgetrennt und anhand ihrer Markierung sichtbar gemacht werden. Aus dem so erhaltenen Muster läßt sich dann leicht die Sequenz der DNA bestimmen.

Ein wesentlicher Nachteil dieser Methode besteht darin, daß zuerst ein Endstück des zu analysierenden DNA-Stranges in seiner Sequenz bestimmt werden muß, um den Primer aufbauen zu können. Ein weiterer Nachteil besteht darin, daß es gewöhnlich sehr schwierig ist, eine ausreichende Menge der zu sequenzierenden DNA zur Verfügung zu stellen.

Diese Schwierigkeiten werden mit der M-13-Phagentemplat-Methode (J. Messing, Methods Enzymol 101, 20-78 (1983)) wesentlich vermindert. Bei dieser Methode wird das doppelsträngige Plasmid des M-13-Phagen als Vektor zur Klonierung der zu sequenzierenden DNA derart verwendet, daß das Plasmid an einer bestimmten Stelle mit der entsprechenden Restriktionsendonuklease aufgeschnitten, in die Öffnung in bekannter Weise die zu sequenzierende DNA insertiert und der so erhaltene Vektor in E.coli kloniert wird. Dabei bildet der E.coli M-13-Phagen, die direkt ins Kulturmedium sezerniert werden und als DNA eine einzelsträngige, circuläre rekombinante M-13-DNA (M-13-ss-DNA) enthalten, in welcher sich die zu sequenzierende DNA befindet. Da die an die Schnittstelle, in die diese DNA insertiert vorliegt, angrenzende Sequenz der Phagen-DNA bekannt ist, kann als Primer stets die dieser bekannten Sequenz komplementäre Sequenz verwendet werden, gleichgültig welche zu sequenzierende DNA in das Plasmid eingefügt wurde. Es kann daher mit einer einzigen Primersequenz eine große Vielzahl von DNA-Strängen sequenziert werden.

Voraussetzung hierfür ist allerdings, daß aus dem vom E.coli sezernierten Phagen die darin enthaltene DNA frei von den sie umhüllenden Proteinen gewonnen wird. Diese Reinigung erfolgte bisher durch Fällung mit Polyethylenglykol, gefolgt von Deproteinisierung durch Extraktionen mit Phenol und Chloroform und schließlich Konzentration der DNA durch Ethanolfällung (M13 Cloning/Dideoxy Sequencing Instruction Manual, Bethesda Research Laboratories 1986).

Dieses Reinigungsverfahren hat den Nachteil, daß es sehr langsam abläuft und sich nicht automatisieren läßt.

Der Erfindung liegt daher die Aufgabe zugrunde, dieses Reinigunsverfahren so zu verbessern, daß diese Nachteile beseitigt werden und das Verfahren nicht nur wesentlich beschleunigt und vereinfacht wird, sondern der Automatisierung zugänglich wird.

Gelöst wird diese Aufgabe erfindungsgemäß durch ein Verfahren der eingangs beschriebenen Art, bei welchem man aus dem Wachstumsmedium der E.coli-Zellen die M-13-Phagen mit Essigsäure fällt, die erhaltene Suspension über Glasfaserfilter filtriert und das Glasfaserfilter mit einer Lösung chaotroper Ionen behandelt und danach die gereinizte DNA vom Glasfaserfilter eluiert.

Zur Durchführung des erfindungsgemäßen Verfahrens werden die E.coli-Bakterien aus dem Wachstumsmedium abzentrifugiert und der gewonnene Überstand mit Essigsäure versetzt. Die verwendete Essigsäuremenge kann in weiten Grenzen variiert werden und Endkonzentrationen zwischen 1 % und 10 % ergaben praktisch gleiche Ausbeuten an gereinigter DNA. Ein besonderer Vorzug der Fällung mit Essigsäure an Stelle von Polyethylenglykol besteht darin, daß die DNA in hoher Ausbeute praktisch ohne Zeitverzug, also durch sofortige Filtration der Suspension nach dem Zusatz der Essigsäure erhalten wird. Daher können Einwirkungszeiten zwischen etwa 0,5 und 30 Minuten angewendet werden, wobei sich mit zunehmender Dauer der Einwirkung die Ausbeute geringfügig erhöht. Nach kurzer Einwirkungszeit, in der Regel 1 bis 5, vorzugsweise etwa 2 Minuten bei normaler Temperatur wird über ein Glasfaserfilter filtriert, zweckmäßig so, daß die Flüssigkeit durchgesaugt werden kann.

In der nächsten Stufe wird die DNA an das Glas im Glasfaserfilter gebunden. Grundsätzlich eignen sich für diese Verfahrensstufe stark chaotrope Ionen, welche eine Dissoziation der Phagenproteine von der Phagen-DNA bewirken können. Die besten Ergebnisse wurden mit NaClO₄-Lösungen erhalten, wobei vorzugsweise eine Konzentration zwischen 3 und 10 M/l, besonders bevorzugt von 4 bis 8 M/l verwendet wird. Gute Ergebnisse wurden auch unter Verwendung von Guanidinium°Hydrochlorid als chaotropes Mittel erhalten.

Die Behandlung mit der Lösung der chaotropen Ionen kann einfach durch Auftropfen auf den Filter und Absaugen erfolgen.

In der bevorzugten Ausführungsform wird der Glasfaserfilter mit einer Perchloratlösung, pH 6,5 bis 8,5, insbesondere pH 7 bis 8 behandelt. Dies erfolgt zweckmäßig durch Auftropfen und Durchsaugen. Besonders bevorzugt wird hierzu eine Lösung, die 4 bis 8 M/l NaClO₄, 5 bis 20 mM/l Tris-HCl, pH 7 bis 8 und 0,5 bis 2 mM/l EDTA enthält, verwendet

Nach dem Entfernen der Chaotrop-, insbesondere der Natriumperchloratlösung wird vorzugsweise mit wäßrigem Ethanol nachgewaschen, zweckmäßig mit 60 bis 80 %igem Ethanol. Nach dem Trocknen der Filter erfolgt dann die Elution in üblicher Weise mit einer verdünnten wäßrigen Salzlösung, wie z.B. in Anal.Biochem. 101, 339-341 (1980) beschrieben. Ein bevorzugtes Elutionsmittel ist 0,5 bis 2 mM/l Tris-HCl, pH 7 bis 8, enthaltend 0,05 bis 0,2 mM/l ETDA, im folgenden als TE bezeichnet. Besonders bevorzugt wird ein pH-Wert von 7,4 bis 7,6.

Ein anderes geeignetes Elutionsmittel sind verdünnte Detergenslösungen, wie z.B. 0,1 % SDS, die jedoch weniger bevorzugt werden.

Ein weiterer Gegenstand der Erfindung ist eine Vorrichtung zur automatisierten Durchführung des erfindungsgemäßen Verfahrens in Form einer Filtrations- und Elutionseinheit. Sie ist gekennzeichnet durch eine Vakuumkammer, die abgedeckt ist mit einer unteren Platte und einer oberen Platte, zwischen denen sich ein Glasfaserfilter befindet. Die untere und obere Platte weisen jeweils eine Vielzahl von miteinander fluchtenden Löchern auf, die durch das Glasfaserfilter geteilt sind. Sowohl die obere als auch die untere Platte besteht vorzugsweise ihrerseits aus einer Sandwich-Struktur, bei der eine elektrisch leitfähige Metallplatte zwischen zwei isolierenden Kunststoffplatten angeordnet ist. Die Metallplatte besteht vorzugsweise aus rostfreiem Stahl. Die beiden Metallplatten in den Verbundplatten weisen Mittel zur Verbindung mit einer Spannungsquelle auf, die es ermöglicht, eine geeignete elektrische Spannung zwischen den beiden Platten anzulegen, um eine Elektroelution durchzuführen. Zwischen der Vakuumkammer und der Verbundkammerdeckplatte kann eine Dichtung, z.B. aus Gummi oder Fett angeordnet sein.

Die Elution kann alternativ auch mittels Mikrotiterzentrifuge erfolgen, wobei der ganze Verbund aus oberer und unterer Platte und Filter in die Zentrifuge gegeben werden kann. Die Mittel zur Verbindung der Metallplatten mit einer Spannungsquelle entfallen dann.

Das erfindungsgemäße Verfahren wird vorzugsweise im Rahmen der DNA-Sequenzierung angewendet. Es läßt sich jedoch auch als rasche und zuverlässige Methode zur Herstellung von M-13-ss -DNA verwenden, wie sie in der Site-gerichteten Mutagenese (gezielte Mutagenese von DNA nach Kramer, Nucl.Acids Res. 12, 9441 (1984)) benötigt wird.

Im folgenden Beispiel wird die Erfindung näher beschrieben.

Dazu wird die erfindungsgemäße Filtrations- und Elutionseinheit verwendet, enthaltend eine Vakuumkammer mit Anschluß zur Vakuumpumpe, die mit einer unteren Kunststoff-Metall-Kunststoffverbundplatte und einer oberen Kunststoff-Metall-Kunststoffverbundplatte, zwischen die ein Glasfaserfilter eingelegt ist, abgedeckt ist. Die-obere und die untere Verbundplatte weisen eine Reihe von Bohrungen auf, die miteinander fluchten und in der oberen Platte trichterförmig, in der unteren Platte zylindrisch ausgebildet sind.

Zwischen den isolierenden Kunststoffplatten ist jeweils eine entsprechende leitfähige Metallplatte angeordnet, die elektrisch so geschaltet werden können, daß die eine Platte als Anode, die andere Platte als Katode wirkt.

Die verwendete Ausführungsform weist 96 Löcher auf und gestattet daher die gleichzeitige Reinigung von 96 verschiedenen M-13-Templaten.

### Beispiel

Es wurde ein M-13 mp 18 rekombinanter Phage verwendet, der ein 800 Basen langes Proteinkinase cDNA-Insert enthält und in Prot.Natl.Acad.Sci. USA 83, 1300-1304 (1986) beschrieben wird. Dieser rekombinante Phage wird erhalten, indem man das Insert aus einem pUC 8-Vektor in die doppelsträngige replikative Form des M-13 mp 18-Phagen insertiert und damit kompetente JM 103 E.coli-Zellen transfiziert. Klare Plaques wurden entnommen und in 2 ml Wachstumsmedium 6 Stunden wachsen gelassen. Dann wurden die Bakterien abzentrifugiert.

1 ml der so erhaltenen Phagensuspension wurde in einem 1,5 ml Zentrifugenröhrchen mit 10 µl Eisessig versetzt, das Röhrchen verschlossen und einmal umgedreht. Nach 2 Minuten bei Raumtemperatur wurde die Suspension auf ein Glasfaserfilter von 7 mm Durchmesser (GF/C der Fa. Whatman Int. Ltd, England) gegeben, welches durch die 7 mm Durchmesser aufweisende Bohrung der unteren und oberen Platten auf einer Filtrations- und Elutionseinheit gemäß Erfindung mit 96 Bohrungen begrenzt ist.

Die Aufgabe der Suspension erfolgte in Aliquots von 0,1 ml. Jedes Aliquot wurde erst aufgegeben, nachdem die vorhergehende Portion durchgesaugt war. Wenn mehrere Proben gleichzeitig behandelt wurden, so erfolgte die Zugabe jeder Probe auf ihr Filter, bevor die nächste Probe auf das nächste Filter aufgebracht wurde.

Anschließend wurde jeder Filter mit insgesamt 1 ml 4 M/l NaClO₄, 10 mM/l Tris-HCl, pH 7,5, 1 mM/l EDTA behandelt, wobei ebenfalls 0,1 ml Aliquots auf den Filter getropft und durchgesaugt wurden.

Anschließend wurde in gleicher Weise mit 1 ml 70 %igem wäßrigem Alkohol gewaschen. Dann wurde die Filtrationseinheit 5 Minuten mit Luft getrocknet und anschließend mit 10 µl TE-Puffer, pH 7,5 unter Anlegung einer Spannung zur Elektroelution eluiert, wobei die Vakuumkammer für die Elution ersetzt wurde durch eine 96 Loch-Mikrotiterplatte, deren einzelne Vertiefungen wieder mit den Bohrungen in den oberen und unteren Verbundplatten fluchteten.

Die so erhaltenen DNA-Präparate wurden als Template für die Sequenzierung sowohl mit radioaktiver Markierung als auch mit Fluoreszenzmarkierung des Primers eingesetzt. Als Enzym wurde das Klenow-Fragment der E.coli-DNA -Polymerase 1 oder T7-DNA-Polymerase verwendet. Dabei ergaben sich mit radioaktiver'Markierung die besten Ergebnisse bei Anwendung von 1 %iger Essigsäurekonzentration und 4 M/l NaClO₄, während mit Fluoreszenzmarkierung die besten Ergebnisse im Bereich von 1 bis 5 % Essigsäure und 4 bis 6 M NaClO₄ erzielt wurden.

## Patentansprüche

1. Verfahren zur Reingewinnung von rekombinanter M-13-ss-DNA durch Fällung des vom E.coli-Wirtsstamm ins Medium ausgeschleusten rekombinanten M-13-Phagen, Abtrennung aus dem Wachstumsmedium und Extraktion der Hüllproteine,
**dadurch gekennzeichnet,**
daß man aus dem Wachstumsmedium die M-13 Phagen mit Essigsäure fällt, die erhaltene Suspension über ein Glasfaserfilter filtriert und das Glasfaserfilter mit einer Lösung chaotroper Ionen behandelt und danach die gereinigte DNA vom Glasfaserfilter eluiert.

2. Verfahren nach Anspruch 1
**dadurch gekennzeichnet,**
daß man als Chaotroplösung eine Perchloratlösung verwendet.

3. Verfahren nach Anspruch 2,
**dadurch gekennzeichnet,**
daß man eine 4 bis 8 M/l Perchloratlösung, pH 6,5 bis 8,5 verwendet.

4. Verfahren nach einem der vorhergehenden Ansprüche,
**dadurch gekennzeichnet,**
daß man zur Fällung das Medium auf eine Endkonzentration zwischen 1 % und 10 % Essigsäure bringt.

5. Verfahren nach einem der vorhergehenden Ansprüche,
**dadurch gekennzeichnet,**
daß man das Glasfaserfilter nach der Behandlung mit der Lösung chaotroper Ionen mit wäßrigem Ethanol wäscht.

6. Verfahren nach einem der vorhergehenden Ansprüche,
**dadurch gekennzeichnet,**
daß man die gereinigte DNA vom Glasfaserfilter mit einer verdünnten wäßrigen Salzlösung eluiert.

7. Verfahren nach Anspruch 6,
**dadurch gekennzeichnet,**
daß man die Elution mit 0,5 bis 2 mM/l Tris-HCl, pH 7 bis 8, enthaltend 0,05 bis 0,2 mM/l EDTA durchführt.

8. Vorrichtung zur Durchführung des Verfahrens der Ansprüche 1 bis 7,
**gekennzeichnet durch**
eine Vakuumkammer, die mit einer Platte abgedeckt ist, über welcher eine weitere Platte angeordnet ist und ein Glasfaserfilter zwischen den beiden Platten, wobei die Platten eine Vielzahl miteinander fluchtender Bohrungen aufweisen und aus einer Sandwich-Struktur bestehen, bei der eine elektrisch leitfähige Metallplatte zwischen zwei isolierenden Kunststoffplatten angeordnet ist.

9. Vorrichtung nach Anspruch 8,
**dadurch gekennzeichnet,**
daß die Metallplatten Mittel zur Verbindung mit einer Spannungsquelle aufweisen.

10. Vorrichtung nach Anspruch 8 oder 9,
**dadurch gekennzeichnet,**
daß die Bohrungen in der oberen Platte trichterförmig, in der unteren Platte zylindrisch ausgebildet sind.

## Claims

1. Process for the obtaining pure of recombinant M 13-ss DNA by precipitation of the recombinant M 13 phages secreted from the E. coli host strain into the medium, separation from the growth medium and extraction of the enveloping proteins, characterised in that one precipitates the M 13 phages from the growth medium with acetic acid, filters the suspension obtained over a glass fibre filter and treats the glass fibre filter with a solution of chaotropic ions and thereafter elutes the purified DNA from the glass fibre filter.

2. Process according to claim 1, characterised in that one uses a perchlorate solution as chaotrope solution.

3. Process according to claim 2, characterised in that one uses a 4 to 8 M/l perchlorate solution, pH 6.5 to 8.5.

4. Process according to one of the preceding claims, characterised in that, for the precipitation, one brings the medium to an end concentration between 1% and 10% of acetic acid.

5. Process according to one of the preceding claims, characterised in that, after the treatment with the solution of chaotropic ions, one washes the glass fibre filter with aqueous ethanol.

6. Process according to one of the preceding claims, characterised in that one elutes the purified DMA from the glass fibre filter with a dilute aqueous salt solution.

7. Process according to claim 6, characterised in that one carries out the elution with 0.5 to 2 mM/l Tris-HCl, pH 7 to 8, containing 0.05 to 0.2 mM/l EDTA.

8. Device for the carrying out of the process of claims 1 to 7, characterised by a vacuum chamber which is covered with a plate over which is arranged a further plate and a glass fibre filter between the two plates, whereby the plates have a plurality of bores aligned with one another and consist of a sandwich structure in which an electrically-conductive metal plate is arranged between two insulating synthetic material plates.

9. Device according to claim 8, characterised in that the metal plate has means for connection with a source of voltage.

10. Device according to claim 8 or 9, characterised in that the bores in the upper plate are formed funnel-shaped , in the lower plate cylindrically.

## Revendications

1. Procédé d'obtention à l'état pur d'ADN sb de M13 recombiné par précipitation du phage M13 recombiné sécrété dans le milieu par la souche hôte E. coli, séparation du milieu de culture et extraction des protéines de l'enveloppe, caractérisé en ce qu'on précipite les phages M13 à partir du milieu de culture avec de l'acide acétique, on filtre la suspension obtenue sur un filtre en fibres de verre, et on traite le filtre en fibres de verre avec une solution d'ions chaotropiques puis on élue l'ADN purifié du filtre en fibres de verre.

2. Procédé selon la revendication 1, caractérisé en ce qu'on utilise comme solution d'agent chaotropique une solution de perchlorate.

3. Procédé selon la revendication 2, caractérisé en ce qu'on utilise une solution de 4 à 8 M/l de perchlorate, de pH 6,5 à 8,5.

4. Procédé selon l'une des revendications précédentes, caractérisé en ce que, pour la précipitation, on amène le milieu à une concentration finale comprise entre 1 % et 10 % d'acide acétique.

5. Procédé selon l'une des revendications précédentes, caractérisé en ce que, après le traitement avec la solution d'ions chaotropiques, on lave le filtre en fibres de verre avec de l'éthanol aqueux.

6. Procédé selon l'une des revendications précédentes, caractérisé en ce qu'on élue l'ADN purifié du filtre en fibres de verre avec une solution saline aqueuse diluée.

7. Procédé selon la revendication 6, caractérisé en ce qu'on effectue l'élution avec 0,5 à 2 mM/l de Tris-HCl, pH 7 à 8, contenant 0,05 à 0,2 mM/l d'EDTA.

8. Dispositif pour la mise en oeuvre du procédé des revendications 1 à 7, caractérisé par une chambre à vide qui est recouverte d'une plaque sur laquelle est disposée une autre plaque et par un filtre en fibres de verre disposé entre les deux plaques, les plaques comportant une multiplicité d'orifices alignés les uns avec les autres et étant constituées d'une structure sandwich qui comprend une plaque métallique conductrice de l'électricité disposée entre deux plaques isolantes de matière plastique.

9. Dispositif selon la revendication 8, caractérisé en ce que les plaques métalliques comportent des moyens de raccordement à une source de tension.

10. Dispositif selon la revendication 8 ou 9, caractérisé en ce que les orifices ont une forme d'entonnoir dans la plaque supérieure, et une forme cylindrique dans la plaque inférieure.
